# EUROPEAN PATENT SPECIFICATION

(11) **EP 1 986 611 B1**
(45) Date of publication and mention of the grant of the patent: **01.05.2013**
(21) Application number: 07750958.6
(22) Date of filing: 15.02.2007
(51) Int. Cl.: A61K 9/16, A61K 9/51, A61K 47/42

(54) **REPEAT SEQUENCE PROTEIN POLYMER NANOPARTICLES OPTIONALLY CONTAINING ACTIVE AGENTS AND THEIR PREPARATION**
WIEDERHOLUNGSSEQUENZPROTEIN-POLYMERNANOTEILCHEN, DIE GEGEBENENFALLS WIRKSTOFFE ENTHALTEN, UND DEREN HERSTELLUNG
NANOPARTICULES DE POLYMERE A SEQUENCE DE REPETITITION PROTEINIQUE CONTENANT EVENTUELLEMENT DES AGENTS ACTIFS ET LEUR PREPARATION

(30) Priority: 23.02.2006 US 776510 P
(43) Date of publication of application: 05.11.2008
(73) Proprietor: Danisco US Inc., Palo Alto, CA 94304-1013 (US)
(72) Inventor: KUMAR, Manoj, Palo Alto, California 94304 (US)
(74) Representative: Kiddle, Simon John
(86) International application number: PCT/US2007/004160
(87) International publication number: WO 2007/100524

(56) References cited:
- WO-A-01/87267
- WO-A-2004/104021

## Description

### FIELD OF THE INVENTION

The present invention relates to nanoparticles formed from repeat sequence protein polymers, and nanoparticles formed from repeat sequence protein polymers and containing active agents that may be released therefrom. The invention also provides for methods for the synthesis of such nanoparticles.

### BACKGROUND OF THE INVENTION

Proteins make up the main structural elements of most organisms, using complex sequences of amino acids that lead to wide arrays of functionalities, including therapeutic proteins and drugs. Delivering protein based therapeutics remains an open challenge, and there is a need to improve the in vivo efficacy of newly developed protein therapeutics by providing better delivery methods and control of antigenic response. One of the most intensely studied structural proteins, Bombyx mori silkworm silk, has generated significant interest because of its remarkable mechanical properties, which rival even spider silk. Elastin, another well-known structural protein, is found predominantly in the body's arterial walls, the lungs, intestines, and skin. Silk elastin like protein (SELP) is a recombinant repeat sequence protein polymer consisting of alternating blocks of silk-like and elastin-like amino acids.

Formation of nanoparticles is known in the art, and the making of dense, hard nanoparticles from metals, metal salts, carbon, plastic and many other dense materials has become an established art. For example, as described in U.S. Pat. No. 6,620,351, nanoparticles of tetracycline, lysozyme, Griseofulein, magnite and fullerine are described, and in some cases coated with the polymer poly(lactide-co-glycolide)(PLGA). Several methods of forming dense, hard nanoparticles are spray drying, milling, fluid energy grinding, electrospray, ultrasound emulsification, solvent evaporation, emulsion polymerization, jet printing, and laser vapor condensation techniques.

However, making less dense, soft nanoparticles from proteins, which contain water and are of a globular nature, makes nanoparticle formation quite challenging and is not described in the art. Making soft nanoparticles from proteins that are less than 500 nm in size has not been reported.

There is a need in the art for developing nanoparticles of structural non-antigenic proteins for *in-vivo* applications, the nanoparticles releasing and delivering protein-based active agents. There is a particular need for the use of such nanoparticles to deliver and controllably release pharmaceutical, neutraceutical, and cosmaceutical proteins, hormones, cytokines and peptides intended for *in vivo* parental delivery. Such delivery may occur through the oral epithelia membrane or by inhaling the nanoparticles through the nasal passages. Such delivery of active agents reduces the amount of agent needed, as compared to parental administration of the active agent alone, and further allows for maintenance of appropriate concentrations of the active agent through the mechanism of controlled release from the nanoparticles.

### SUMMARY OF THE INVENTION

The invention is directed to nanoparticles comprising one or more repeat sequence protein polymers, and nanoparticles comprising one or more repeat sequence protein polymers and containing one or more active agents , wherein the nanoparticles have at least one dimension smaller than 600 nanometers.

A first embodiment of the invention is a repeat sequence protein polymer nanoparticle. In a more specific embodiment of the invention the nanoparticle is a co-polymer comprising sequences derived from silk and elastin like protein, termed SELP. In yet another specific embodiment of the invention the nanoparticle is a co-polymer derived from silk and collagen like protein. In another embodiment of the invention, a nanoparticle of a repeat sequence protein polymer contains an active agent, such as one or more nutrients, proteins or peptides, hormones, drugs, or pharmaceutical agents.. In yet a more specific embodiment, a SELP nanoparticle contains an active agent. In an additional embodiment, a SELP nanoparticle contains insulin or interferon.

The nanoparticles of the present invention may retain variable percentages of water, or other solvents used to make the nanoparticles as well as other additives selected to tailor properties of the nanoparticles.

This invention also extends to methods for the formation of repeat sequence protein polymer nanoparticles, and repeat sequence protein polymer nanoparticles containing active agents.

One method of the present invention comprises the use of supercritical antisolvent precipitation with enhanced mass transfer (SAS-EM). The SAS-EM method uses super-critical fluids and vibrational atomization where a dispersion jet is deflected by a vibrating surface into droplets. The size of the nanoparticles is controlled by selecting the vibration intensity of the deflecting surface.

Other methods for making less dense, soft nanoparticles may be used and include, for example, other supercritical fluids methods such as supercritical antisolvent (SAS) precipitation, the rapid expansion of supercritical solutions (RESS) technique, or any other particle method suitable from making less dense, soft nanoparticles.

The size and composition of SELP material added to the nanoparticle may be selected to provide a nanoparticle with desired controlled release, or degradation properties.

### BRIEF DESCRIPTION OF THE DRAWINGS

Figure 1 is an SEM micrograph showing nanoparticles of SELP47K.
Figure 2 is an SEM micrograph showing nanoparticles of SELP27K.
Figure 3 is an SEM micrograph showing nanoparticles of SELP67K.
Figure 4 is an SEM micrograph showing nanoparticles of SELP67K containing the active agent insulin.

### DETAILED DESCRIPTION OF THE INVENTION

The present invention is directed to compositions that are repeat sequence protein polymer nanoparticles. The invention further is directed to repeat sequence protein polymer nanoparticles that include one or more active agents. In one embodiment of the invention, a nanoparticle includes an active agent that is a hormone, for instance, insulin, or a cytokine, and the repeat sequence protein polymer is a co-polymer having sequences derived from silk and elastin, termed SELP. The nanoparticles of the present invention are exfoliated materials produced under controlled conditions.

The invention further includes methods for the formation of nanoparticles and the formation of nanoparticles that comprise an active agent and a repeat sequence protein polymer. One method used s SAS-EM, which produces particles within the nanometer size range and having a narrow size distribution. This method utilizes a vibrational technology that is controllable to select nanoparticle size.

### DEFINITIONS

For purposes of this invention, the following definitions shall apply:
"Active agent" means one or more of proteins, peptides, hormones, drugs, cytokines, and any other therapeutic, cosmaceutic, neutraceutic agent suitable for inclusion in a nanoparticle destined for parental or nasal administration to a human or animal. Protein active agents may be, but are not limited to enzymes, for example any protolytic enzyme, hydrolase, glucose oxidase, asparaginase, urokinase, ribonuclease, hyaluronidase, trypsin, streptokinase, beta lactamase, lysozyme, collagenase, glutaminase, chymotrypsin, papain, deoxyribonuclease, and uricase. Hormones may be, but are not limited to, growth factors, ACTH, aldosterone, renin, cortisol, DHEASE, estrogen, growth hormone, IGF-1, luteinizing hormone, prolactin, testosterone, and thyroid-stimulating hormone. Cytokines may be, but are not limited to, interferon and chemokines. Neutraceuticals may be, but are not limited to, taurine, D-ribose, gamma-oryzanol, glucosamine, D-glucouronolactone, creatine monohydrate, coenzyme Q10, Vitamin C, St. John's wort, carnitine, and caffeine monohydrate. Drugs may be, but are not limited to Celebrex™, cancer treatment agents, betaferon, nicotine, vaccines, acetaminophen, lidocaine, fentamyl, diabetic management agents, and antifungal agents. Peptides may be, but are not limited to antimicrobial peptides, such as protegrin, indolicidin; antiviral peptides; antifungal peptides, ACTH, alpha-MSH, corticastatin, amylin, biotin angiotensin, rennin, bombesin, osteocalcin, myelopeptide, endomorphin, AVEGF, and gastrin. It will be recognized by those skilled in the art that mixtures of active agents may be used.
"Nanoparticle" means a microscopic particle whose size is measured in nanometers. (ie. at least one dimension is smaller than 600 nanometers, smaller than 500 nanometers, smaller than 400 nanometers, smaller than 300 nanometers, smaller than 200 nanometers, smaller than 100 nanometers, and smaller than 50 nanometers.
"Release" means that an active agent may be controllably separated from a nanoparticle incorporating the active agent. Nanoparticles of repeat sequence protein polymers retain the active agent(s) in the nanoparticle by either electrostatic charges or by hydrogen bonding, or hydrophobic interaction. Release of the active agent may be accomplished by interrupting the charge or hydrophobic interaction, or by controlled release over time through degradation, dispersion, or dissolving of the nanoparticle of repeat sequence protein polymer that allows release of the active agent(s) from the nanoparticle.

Without wishing to be bound by any particular theory, it is believed that the active agent is retained within the repeat sequence protein polymer by either electrostatic charges or by hydrogen bonding, or hydrophobic interaction. Release of the active agent may be accomplished by interrupting the charge or hydrophobic interaction, or by controlled release over time through degradation of the repeat sequence protein polymer.

Specifically, anionic charged active agents can be held in SELP based nanoparticles by electrostatic interaction because of the cationic nature of SELP. Hydrophobic therapeutic and cosmaceutical active agents can be held inside SELP based nanoparticles because of hydrophobic interactions.

Controllable release mechanisms include, for example, diffusion through a rate controlling media, erosion of biodegradable repeat sequence polymer nanoparticles, shearing of the nanoparticle, or a combination of diffusion and erosion or shearing. Controlled release further includes triggered release, which occurs in the presence of external conditions, such as heat, pressure, electric fields, pH, salt concentrations, ionic strength, and solvents, as described in, for example, WO 04/104020. The repeat sequence protein polymer may act as a rate-controlling polymer. The release rate of the active agents from suitable repeat sequence protein polymers may be altered by modifying the composition and sequence of the repeat sequence protein polymer and by modifying the size and geometry of the repeat sequence protein polymer with which the active agent has been complexed.

### REPEAT SEQUENCE PROTEIN POLYMERS

The repeat sequence protein polymer (RSPP) can be any polypeptide with at least one distinct domain repeated throughout the entire sequence two or more times.

The at least two distinct repeating domains of the RSPPs suitable for the present invention may be derived from a natural, chemically synthesized and/or modified, recombinant protein, or mixtures thereof. For example, the repeating sequence units may be derived from natural structure supporting materials such as silk, elastin, and collagen. Alternatively, the repeating sequence units may be derived from synthetic structures.

One skilled in the art will appreciate the various naturally occurring proteins containing repeating sequence units, which can be used for designing and producing the repeat sequence protein polymers of the present invention, any of which may be employed herein. Specifically, there are more than six hundred repeating amino acid sequence units known to exist in biological systems. The natural OR synthetic protein repeating amino acid sequence units are derived from elastin, collagen, abductin, byssus, extensin, flagelliform silk, dragline silk, gluten high molecular weight subunit, titin, fibronectin, leminin, gliadin, glue polypolypeptide, ice nucleating protein, keratin mucin, RNA polymerase II, resalin or a mixture thereof.

RSPP repeating sequence units for the natural or synthetic materials listed above are described and the amino acid sequences are shown in WO 04080426A1, which is incorporated herein in its entirety.

The repeat sequence protein polymer (RSPP) formula comprises:

Ty[(Aₙ)ₓ(B)_{b}(A'ₙ')ₓ'(B')_{b}'(A"ₙ")ₓ"]ᵢ'T'_{y}'

wherein: T and T' each comprise an amino acid sequence of from about 1 to about 100 amino acids, wherein the amino acid sequence of T' is the same as or different from the amino acid sequence of T; y and y' are each an integer from 0 to 1, wherein the integer of y' is the same as or different from the integer of y; A, A' and A" are each individual repeating amino acid sequence units comprising from about 3 to about 30 amino acids, wherein the amino acid sequence of A' and the amino acid sequence of A" are the same as or different from the amino acid sequence of A; n, n', and n" are each integers of at least 2 and not more than 250; x, x' and x" are each 0 or an integer of at least 1, wherein each integer varies to provide for at least 30 amino acids in the A', A' and A" individual amino acid sequence repeating units, and wherein the integer of x' and the integer of x" are the same as or different from the integer of x and x, x', and x" cannot all be zero; B and B' each comprise an amino acid sequence of from about 4 to about 50 amino acids, wherein the amino sequence of B' is the same as or different from the amino acid sequence of B; b and b' are each an integer from O to 3, wherein the integer of b' is the same as or different from the integer of b; and i is an integer from 1 to 500.

The repeating amino acid sequence units may comprise identical repeating sequence units or may comprise different repeating sequence unit combinations, which join together to form a block copolymer or an alternating block copolymer. Additionally, the individual repeating amino acid sequence units of the repeat sequence protein polymer comprise from about 3 to about 30 amino acids or from about 3 to about 8 amino acids. Moreover, the same amino acid may appear at least twice in the same repeating sequence unit.

It will be further understood by those having skill in the art that the repeat sequence protein polymers of the present invention may be monodispersed or polydispersed. For purposes of defining and describing the present invention, "monodispersed" polymers are polymers having a single defined molecular weight. For purposes of defining and describing the present invention, "polydispersed" polymers are polymers that have been subjected to proteolysis or other means of subdivision, or were produced or modified in such a manner as to give rise to a distribution of molecular weights.

In one embodiment, the copolymers are combinations of silk units and elastin units to provide silk-elastin copolymers having properties distinctive from polymers having only the same monomeric unit.

A silk-elastin polymer, SELP47K, may be used as the repeat sequence protein polymer of the present invention. The SELP47K is a homoblock protein polymer that consists exclusively of silk-like crystalline blocks and elastin-like flexible blocks. SELP47K is 70% proline, valine, and alanine, and has hydrophobic characteristics. The repeat sequence protein polymer may also comprise SELP 47-E13, SELP 47R-3, SELP 47K-3, SELP 47 E-3, SELP 67K, and SELP 58.

In one embodiment of the invention, the structure of the silk elastin-like protein is *Head-*(*S₂E₃E_{K}E₄S₂*)₁₃-*Tail,* where S is the silk-like sequence of amino acids GAGAGS, *E* is the elastin-like sequence GVGVP, and *E_{K}* is the elastin like sequence modified with a lysine residue GKGVP. The head sequence of amino acids is MDPVVLQRRD WENPGVTQLN RLAAHPPFAS DPM and the tail sequence is AGAGSGAGAM DPGRYQDLRS HHHHHH. The copolymer contains 886 amino acids, with 780 amino acids in the repeating sequence unit. The SELP47K has a molecular weight of about 70,000 Daltons, and a pI of 10.5.

In another embodiment, the structure of the silk-elastin-like protein, SELP67K, is represented by the following formula; Head (S₃E₃EₖE₄S₃)₁₃ Tail, where S is the silk-like sequence of amino acids GAGAGS, E is the elastin-like sequence GVGVP, and Eₖ is the elastin like sequence modified with a lysine residue GKGVP. The head sequence of amino acids is MDPVVLQRRD WENPGVTQLN RLAAHPPFAS DPM and the tail sequence is AGAGSGAGAM DPGRYQDLRS HHHHHH. The copolymer contains 1042 amino acids, with 936 amino acids in the repeating sequence unit. SELP67K has a molecular weight of about 80,347 Daltons, and a pI of 10.53.

The properties of a number of SELP variants are shown below in Table 1.

**Table 1. SELP variants, properties.**

| Variant Name | Number of Subunits | Lysine Substitution | Molecular Weight (Da) | Isoelectric Point |
|---|---|---|---|---|
| SELP47E | 13 | Glutamic Acid | 70,212 | 4.16 |
| SELP47K-3 | 3 | none | 20,748 | 9.52 |
| SELP47R-3 | 3 | Arginine | 20,960 | 10.5 |
| SELP47E-3 | 3 | Glutamic Acid | 20,879 | 5.9 |
| SELP27K | 13 | none | 59,401 | 10.53 |
| SELP37K | 13 | none | 64,605 | 10.53 |
| SELP58 | 13 | none | 74,765 | 6.7 |
| SELP67K | 13 | none | 80,347 | 10.53 |

One skilled in the art will appreciate the various methods for producing the repeat sequence protein polymers of the present invention, any of which may be employed herein. For example, the repeat sequence protein polymer may be produced by generally recognized methods of chemical synthesis, for example, L Andersson et al., Large-scale synthesis of peptides, Biopolymers 55(3), 227-50 (2000)); genetic manipulation (for example, J. Cappello, Genetically Engineered Protein Polymers, Handbook of Biodegradable Polymers, Domb, A.J.; Kost, J.; Wiseman, D. (Eds.), Harvard Academic Publishers, Amsterdam; pages 387-414); and enzymatic synthesis (for example, C.H. Wong & K.T. Wang, New Developments in Enzymatic Peptide Synthesis, Experientia 47(11-12), 1123-9 (1991)). For example, the repeat sequence protein polymers of the present invention may be produced using the methods described in U.S. Patent Nos. 5,243,038; 6,355,776; and WO 07080426A1. In another example, the repeat sequence protein polymers may be produced utilizing non-ribosomal peptide synthase (for example, H.V.Dohren, et al., Multifunctional Peptide Synthase, Chem. Rev. 97, 2675-2705(1997).

The E. coli strains containing a specific silk-elastin repeat sequence protein copolymer SELP47K, SELP37K and SELP27K recombinant DNA were also obtained from Protein Polymer Technologies, Inc. of San Diego, California. SELP67K, SELP58, SELP37K and SELP27K variant proteins were produced in 14 L fed batch culture using standard SELP47K production protocols, as described above. Proteins were purified and characterized as follows: 40 grams of cell pastes collected from 14L cultures were lysed via French-press followed by the addition of polyethyleneimine (0.8 w/v%). Centrifugation was used to separate the cellular debris from the cell extract. SELP polymers were precipitated from the cell extract using ammonium sulfate (30% saturation), collected by centrifugation and reconstituted in water.

The protocol used for the genetic engineering of variants SELP47E, SELP47K-3, SELP47R-3, and SELP47E-3 is a modification of a commercially available kit designed to create single base pair changes in multiple sites along a particular DNA sequence (QUIKCHANGE^{®} Multi (Site-Directed Mutagenesis Kit), Stratagene cat #200513). The standard protocol involves the construction of single direction 5' phosphorylated primers that will hybridize to plasmid template regions of interest and incorporate point mutations. Thermocycling is employed that includes a ligation reaction designed to link the multiple primers during each round of synthesis.

### METHODS FOR FORMING NANOPARTICLES

The method for forming nanoparticles of the present invention may be any method suitable for repeat sequence protein polymers. One such method is the Supercritical Antisolvent Precipitation with Enhanced Mass Transfer (SAS-EM) technique, as described in U.S. Pat. No. 6,620,351. The SAS-EM technique uses supercritical fluid as an antisolvent. Unlike conventional SAS techniques, the SAS-EM method deflects the dispersion jet with a vibrating surface, and the deflected jet is atomized into micro-droplets that spread evening on the vibrating surface thereby forming a film. Wavelets caused by the vibrating surface are formed and increase in amplitude until the tip of the wavelet breaks off as droplets emitted into the supercritical fluid. CO₂ is rapidly transferred into the droplets while the solvent is transferred out of the droplets. The material precipitates as fine particles. This method is described more fully in U.S. Pat. No. 6,620,351, which is incorporated herein in its entirety. The method may be used for particles that are not suitable for nanoparticle formation using the SAS method.

Other methods for making less dense, soft nanoparticles may be used and include, for example, other supercritical fluids methods such as supercritical antisolvent (SAS) precipitation, the rapid expansion of supercritical solutions (RESS) technique, spray freezing in liquid nitrogen, and emulsion polymerization.

The nanoparticles may retain variable amounts of the water or other solvents used to make the composites. For instance, the nanoparticles may retain from about 0.1% to about 90%, about 1% to about 50%, about 1% to about 25%, about 1% to about 15%, about 1% to about 10%, about 5% to about 20%, and about 5% to about 10% of water or other solvents. In one embodiment, DSMO is used as a solvent without retention of the solvent in the formed nanoparticle.

The nanoparticles may include additives to tailor and vary properties. For instance, additives may be, but are not limited to, polyethylene glycols, polylactides, gelatin, polyoxomers such as Pluonics™ (BASF), lactine, vegetable oils such as cottonseed oil and soybean oil, human serum albumin, hardening agents such as glutaraldehyde, and multivalent ions such as calcium.

The following examples are included to illustrate embodiments of the invention and are not intended to be limiting thereof.

### EXAMPLES

### Example 1

### Production of Silk-elastin like protein (SELP).

Monodispersed silk-elastin protein polymer SELP47K and SELP67K were produced by fermenting a recombinant E. coli strain to produce a cell-paste containing monodispersed SELP47K and SELP67k as described in US2004/0180027A1. The cell-paste is placed in ice-cold water and homogenized to make the cell extract. The cell-extract is mixed with polyethyleneimine and a filter-aid and allowed to sit at 7°C for one hour. The polyethyeleneimine causes precipitation of cell debris and a significant amount of *E. coli* proteins. The SELP47K and SELP67k containing reaction mixtures were then filtered using a Rotary Drum Vacuum Filter (RVDF). The filtered SELP47K and SELP67k solutions were then mixed with ammonium sulfate to 25% saturation, which leads to precipitation of the SELP materials. Precipitated SELP47K and SELP67k and mother liquor are mixed with a filter-aid and again filtered using RVDF. The RVDF cake containing SELP47K and SELP67k and filter-aid are mixed with cold water to dissolve the SELP47K and SELP67k. This precipitation and solubilization step is repeated to improve the purity profile. Purified monodispersed SELP47K and SELP67k are then water-exchanged until the conductivity of SELP solution reached 50 µS/cm². The monodispersed SELP solution was then concentrated to 10% wt/vol and then lyophilized to make powdered monodispersed SELP47K and SELP67k protein polymer. The material was stored at -70°C until needed for application testing.

### Example 2

### Preparation of Nanoparticle Formation of SELP.

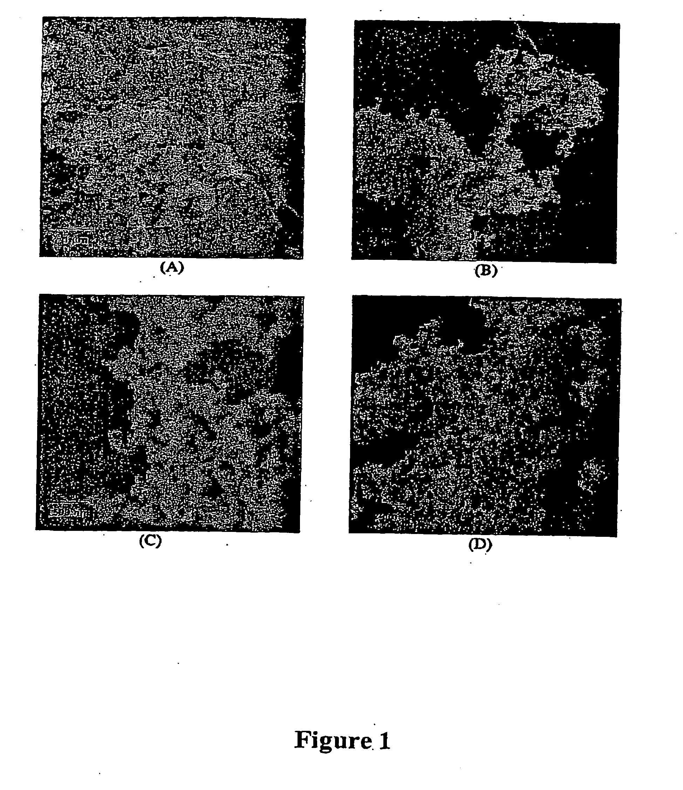

SELP47K,SELP67k, and SELP27k nanoparticles were formed using the supercritical antisolvent precipitation with enhanced mass transfer (SAS-EM) method of Gupta and Chattopadhyay (US Pat. No. 6,620,351). Figure 1 of the '351 patent includes a schematic of the SAS-EM apparatus, the main component of which is a high-pressure precipitation cell with an approximate total volume of 110 cm³. A titanium horn from H, Sonics and Materials, Inc. and having a tip that is 1.25 cm in diameter is attached to the precipitations cell to provide an ultrasonic field generated by a 20 kHz ultrasonic processor having a maximum power of 600 Watt (U, Sonics and Materials, Inc.). For this experiment, the horn was operated at 60 watts. The volume of the precipitation cell with the horn in place is approximately 80 cm³, and the ultrasonic processor delivers ultrasound at constant amplitude. The experiment was carried out at constant temperature and constant pressure of the supercritical CO₂ medium (31°C and 1071 psi). To maintain these supercritical conditions, the experiments were carried out at 40°C and 1500 psi, and the constant pressure was maintained using an ISCO syringe pump (Model 500D) to continuously deliver CO₂ at constant pressure, constant temperature using a heating coil. The syringe pump is operated with a RTD controller device and includes a temperature sensor.

CO₂ was introduced into the precipitation cell through the inlet port at the bottom of the vessel and purged through an inline membrane filter, to prevent loss of active agent, from the exit at the top of the vessel. The precipitation cell was allowed to reach steady temperature and pressure conditions such that the CO₂ was under supercritical conditions. After adjusting the ultrasonic intensity to the desired value, filtered SELP47K (Genencor International, Inc. Palo Alto, CA) solution in DIMETHYL SULFOXIDE (DMSO) or SELP67k, both made according to the method described in Example 1, was injected through a PEEK nozzle (100 um internal diameter) into the precipitation cell at a rate of 1 ml/minute with a manual syringe pump. During injection, CO₂ was continuously purged to allow DMSO extracted by the CO₂ to be removed from the system. Because the nozzle is horizontal to the horn and touches the center of the horn, the liquid injected undergoes maximum ultrasonic intensity, and the liquid jet breaks into very fine droplets. The diameter of the droplets is controlled by the ultrasonic vibration. Next the ultrasound was terminated and the system was then purged with fresh CO₂ at about 5 times the volume of the precipitation cell to completely remove DMSO. The cell was then depressurized to collect the nanoparticles.

Small SELP67k nanoparticles were obtained, with some of the nanoparticles in the 100-nanometer size range. SELP47k and SELP27k nanoparticles were in the 200 nanometer to 500-nanometer size range. The morphology of the SELP47k nanoparticles was spherical and monodispersed. The SELP27k nanoparticles were less spherical and aggregated more than the SELP47k nanoparticles. The SELP67k nanoparticles were spherical and showed some bridging between nanoparticles.

### Example 3

### Nanoparticle formation of SELP67k with the active agent insulin.

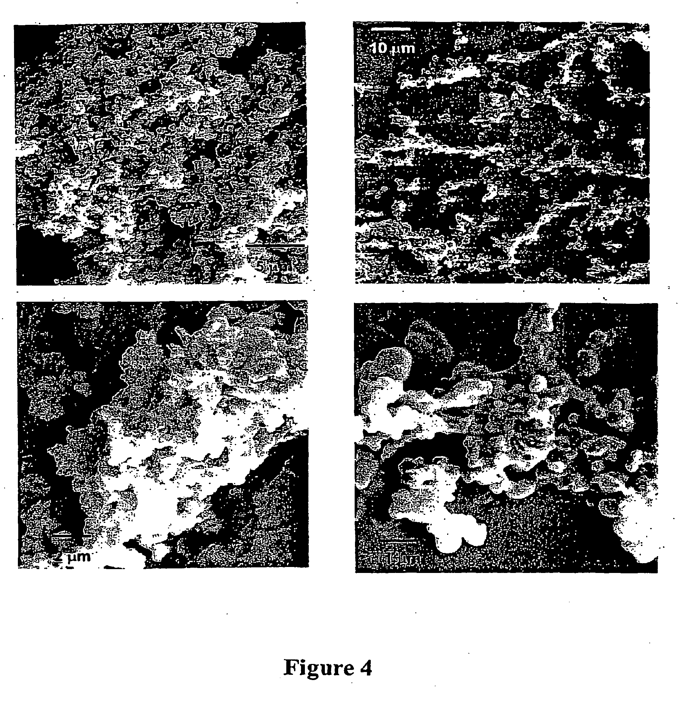

DMSO was selected as a solvent for SELP67k to dissolve the polymer to make nanoparticles using a solvent screening method. Insulin also has good solubility in DMSO. The SELP67k and insulin (Sigma) were simultaneously dissolved in the DMSO at a mass ratio of 9:1. The total protein polymer plus insulin concentration was about 2.5 mg/ml in DMSO. 5 ml of this solution was injected into supercritical carbon dioxide precipitation at 1500 psi and 40°C with 60W of ultrasound power for enhanced mixing. The solution was injected using a 100 uM diameter, 12 cm long nozzle by pressuring at 3000 psi. (See example 2 for details of the procedure). Figure 4 is SEM micrographs of the resulting nanoparticles at different magnifications. The morphology of the SELP67K/insulin particles is similar to the morphology of the pure SELP67K nanoparticles of Example 2. Some fusing of particles was observed at the 100 um size range. The insulin nanoparticles were generally needle shaped nanoparticles with the insulin encapsulated in the SELP67K repeat sequence protein polymer.

### Example 4:

### Particle Size Analysis

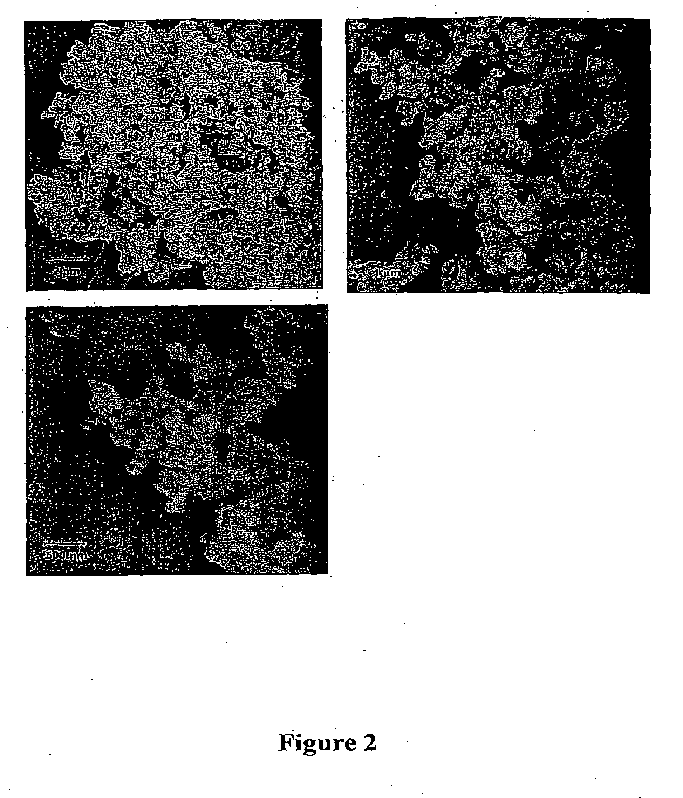

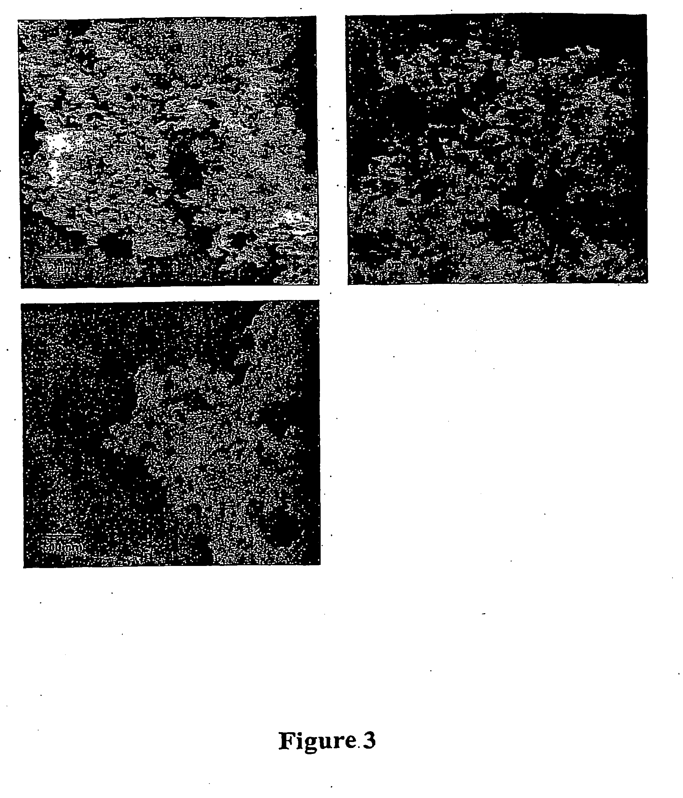

The SELP nanoparticles obtained according to Example 1 were analyzed for size and surface morphology using a scanning electron microscope (SEM; Zeiss, Model DSM 940). The nanoparticles were spread out upon a carbon tape that was fastened to an aluminum stub. Then the particles were coated with gold using a Sputter Coater^{®} (Electron Microscopy Systems, Model EMS 550) under a vacuum of 0.2 mbar. The sputtering process applies vacuum of 0.1 mbar to the nanoparticles in a closed chamber. The particles are then coated with gold vapor in the presence of argon gas at 0.2 mbar. The particles were coated twice at one-minute intervals to protect against exposing the protein to excessive heat. The gold-coated layer(s) conducts electrons in the SEM. The nanoparticles were then observed under SEM and micrographs were recorded. The results are shown in Figures 1, 2, and 3 for SELP47k, SELP27k and SELP67k. In the figures, the nanoparticles are spherical and range in size between about 100nm to about 350 nm.

## Claims

1. A nanoparticle comprising at least one repeat sequence protein polymer, wherein the nanoparticle has at least one dimension smaller than 600 nanometers.

2. The nanoparticle of claim 1, wherein a formula of the repeat sequence protein polymer comprises:
**T_{y}[(Aₙ)ₓ(B)_{b}(A'_{n'})_{x'}(B')_{b'}(A"ₙ")ₓ.]ᵢT'_{y'}**
wherein:
T and T' each comprise an amino acid sequence of from 1 to 100 amino acids, wherein the amino acid sequence of T' is the same as or different from the amino acid sequence of T;
y and y' are each an integer from 0 to 1, wherein the integer of y' is the same as or different from the integer of y;
A, A' and A" are each individual repeating sequence units comprising from 3 to 30 amino acids, wherein the amino acid sequence of A' and the amino acid sequence of A" are the same as or different from the amino acid sequence of A;
n, n' , and n" are integers of at least 2 and not more than 250;
x, x' and x" are each 0 or an integer of at least 1, wherein each integer varies to provide for at least 30 amino acids in the A, A' and A" individual repeating sequence units, and wherein the integer of x' and the integer of x" are the same as or different from the integer of X;
B and B' each comprise an amino acid sequence of from about 4 to about 50 amino acids, wherein the amino sequence of B' is the same as or different from the amino acid sequence of B;
b and b' are each an integer from 0 to 3, wherein the integer of b' is the same as or different from the integer of b; and
i is an integer from 1 to 100.

3. The nanoparticle of claim 2, wherein the at least one repeat sequence protein polymer is a copolymer.

4. The nanoparticle of claim 3, wherein the copolymer is a combination of silk units and elastin units.

5. The nanoparticle of any one of claims 1 to 4, further comprising an active agent selected from proteins, peptides, hormones, drugs, cytokines, therapeutics, cosmaceuticals and neutraceuticals.

6. The nanoparticle of claim 5, wherein the active agent is a hormone.

7. The nanoparticle of claim 6, wherein the hormone is selected from insulin, growth factors, ACTH, aldosterone, renin, cortisol, DHEASE, estrogen, growth hormone, IGF-l, luteinizing hormone, prolactin, testosterone, and thyroid-stimulating hormone.

8. The nanoparticle of claim 7, wherein the hormone is insulin.

9. The nanoparticle of any one of the preceding claims further comprising water or a solvent and/or an additive selected from polyethylene glycols, polylactides, gelatin, polyoxomers, lactine, vegetable oils, human serum albumin, hardening agents, and multivalent ions.

10. The nanoparticle of any one of the preceding claims having a size of from 100 nanometers to 500 nanometers.

11. The nanoparticle of any one of claims 5 to 10, wherein the active agent is releasable retained within the repeat sequence protein polymer by electrostatic charges, hydrogen bonding, or hydrophobic interaction.

12. A nanoparticle of any one of the preceding claims for use in a nasal or parentally administratable nutraceutical, cosmaceutical or therapeutical composition.

13. A composition comprising nanoparticles according to any one of claims 1 to 11.

14. A method for making a nanoparticle according to any one of claims 1 to 11, the method comprising:
providing at least one repeat sequence protein polymer;
subjecting the at least one repeat sequence protein polymer to a technique selected from Supercritical Antisolvent Precipitation with Enhanced Mass Transfer (SAS-EM), supercritical antisolvent (SAS) precipitation, rapid expansion of supercritical solutions (RESS), spray freezing in liquid nitrogen, and emulsion polymerisation.

15. The method of claim 14, wherein providing at least one repeat sequence protein polymer comprises providing a copolymer and the step of subjecting comprises subjecting the copolymer to SAS-EM.

16. The method of claim 14 further comprising adding an active agent to the copolymer prior to subjecting the copolymer to SAS-EM and/or adding a hormone to the copolymer.

## Patentansprüche

1. Nanoteilchen umfassend mindestens ein Wiederholungssequenz-Proteinpolymer, wobei das Nanoteilchen mindestens eine Dimension aufweist, die kleiner als 600 Nanometer ist.

2. Nanoteilchen nach Anspruch 1, wobei eine Formel des Wiederholungssequenz-Proteinpolymers
T_{y}[(Aₙ)ₓ(B)_{b}(A'_{n'})_{x'}(B')_{b'}(A"ₙ")_{x'}]iT'_{y'}
umfasst, wobei:
T und T' jeweils eine Aminosäuresequenz von 1 bis 100 Aminosäuren umfassen, wobei die Aminosäuresequenz von T' gleich wie die oder verschieden von der Aminosäuresequenz von T ist;
y und y' jeweils eine ganze Zahl von 0 bis 1 sind, wobei die ganze Zahl von y' gleich wie die oder verschieden von der ganzen Zahl von y ist;
A, A' und A" jeweils einzelne Wiederholungssequenzeinheiten sind, die 3 bis 30 Aminosäuren umfassen, wobei die Aminosäuresequenz von A' und die Aminosäuresequenz von A" gleich wie die oder verschieden von der Aminosäuresequenz von A sind;
n, n' und n" ganze Zahlen von mindestens 2 und nicht mehr als 250 sind;
x, x' und x" jeweils 0 oder eine ganze Zahl von mindestens 1 sind, wobei jede ganze Zahl unterschiedlich ist, um mindestens 30 Aminosäuren in den einzelnen A-, A'- und A"-Wiederholungssequenzeinheiten bereitzustellen, und wobei die ganze Zahl von x' und die ganze Zahl von x" gleich wie die oder verschieden von der ganzen Zahl von X sind;
B und B' jeweils eine Aminosäuresequenz von etwa 4 bis etwa 50 Aminosäuren umfassen, wobei die Aminosäuresequenz von B' gleich wie die oder verschieden von der Aminosäuresequenz von B ist;
b und b' jeweils eine ganze Zahl von 0 bis 3 sind, wobei die ganze Zahl von b' gleich wie die oder verschieden von der ganzen Zahl von b ist; und
i eine ganze Zahl von 1 bis 100 ist.

3. Nanoteilchen nach Anspruch 2, wobei das mindestens eine Wiederholungssequenz-Proteinpolymer ein Copolymer ist.

4. Nanoteilchen nach Anspruch 3, wobei das Copolymer eine Kombination von Seideneinheiten und Elastineinheiten ist.

5. Nanoteilchen nach einem der Ansprüche 1 bis 4, des Weiteren einen Wirkstoff umfassend ausgewählt unter Proteinen, Peptiden, Hormonen, Arzneimitteln, Cytokinen, therapeutischen Mitteln, Cosmeceuticals und Neutraceuticals.

6. Nanoteilchen nach Anspruch 5, wobei der Wirkstoff ein Hormon ist.

7. Nanoteilchen nach Anspruch 6, wobei das Hormon unter Insulin, Wachstumsfaktoren, ACTH, Aldosteron, Renin, Cortisol, DHEASE, Östrogen, Wachstumshormon, IGF-1, luteinisierendem Hormon, Prolactin, Testosteron und schilddrüsenstimulierendem Hormon ausgewählt wird.

8. Nanoteilchen nach Anspruch 7, wobei das Hormon Insulin ist.

9. Nanoteilchen nach einem der vorhergehenden Ansprüche, des Weiteren Wasser oder ein Lösungsmittel und/oder ein Zusatzmittel umfassend ausgewählt unter Polyethylenglykolen, Polylactiden, Gelatine, Polyoxomeren, Lactin, Pflanzenölen, menschlichem Serumalbumin, Härtungsmitteln und mehrwertigen Ionen.

10. Nanoteilchen nach einem der vorhergehenden Ansprüche, das eine Größe von 100 Nanometern bis 500 Nanometern aufweist.

11. Nanoteilchen nach einem der Ansprüche 5 bis 10, wobei der Wirkstoff freisetzbar innerhalb des Wiederholungssequenz-Proteinpolymers durch elektrostatische Ladungen, Wasserstoffbindung oder hydrophobe Wechselwirkung zurückgehalten wird.

12. Nanoteilchen nach einem der vorhergehenden Ansprüche zur Verwendung in einem/einer nasal oder parenteral verabreichbaren Nutraceutical, Cosmeceutical oder therapeutischen Zusammensetzung.

13. Zusammensetzung umfassend Nanoteilchen nach einem der Ansprüche 1 bis 11.

14. Verfahren zur Herstellung eines Nanoteilchens nach einem der Ansprüche 1 bis 11, wobei das Verfahren Folgendes umfasst:
das Bereitstellen mindestens eines Wiederholungssequenz-Proteinpolymers;
das Unterwerfen des mindestens einen Wiederholungssequenz-Proteinpolymers einer Technik ausgewählt unter Ausfällung durch superkritisches Antilösungsmittel mit verbesserter Massenübertragung (SAS-EM), Ausfällung durch superkritisches Antilösungsmittel (SAS), Schnellausdehnung superkritischer Lösungen (RESS), Sprühgefrieren in flüssigem Stickstoff und Emulsionspolymerisation.

15. Verfahren nach Anspruch 14, wobei das Bereitstellen von mindestens einem Wiederholungssequenz-Proteinpolymer das Bereitstellen eines Copolymers umfasst und der Schritt des Unterwerfens das Unterwerfen des Copolymers SAS-EM gegenüber umfasst.

16. Verfahren nach Anspruch 14, des Weiteren das Hinzusetzen eines Wirkstoffs zu dem Copolymer vor dem Unterwerfen des Copolymers SAS-EM gegenüber und/oder dem Hinzusetzen eines Hormons zu dem Copolymer umfassend.

## Revendications

1. Nanoparticule comprenant au moins un polymère de protéine à séquence de répétition, dans laquelle la nanoparticule a au moins une dimension inférieure à 600 nanomètres.

2. Nanoparticule selon la revendication 1, dans laquelle une formule du polymère de protéine à séquence de répétition comprend:
T_{y}[(Aₙ)ₓ(B)_{b}(A'_{n'})ₓ,(B')_{b'}(A"ₙ")ₓ,]iT'_{y'}
dans laquelle:
T et T' comprennent chacun une séquence d'acides aminés de 1 à 100 acide(s) aminé(s), dans laquelle la séquence d'acides aminés de T' est identique ou différente de la séquence d'acides aminés de T;
y et y' sont chacun un nombre entier d'une valeur de 0 à 1, dans laquelle le nombre entier de y' est identique ou différent du nombre entier de y;
A, A' et A" sont chacun des motifs de séquence de répétition individuels comprenant de 3 à 30 acides aminés, dans laquelle la séquence d'acides aminés de A' et la séquence d'acides aminés de A" sont identiques ou différentes de la séquence d'acides aminés de A;
n, n' et n" sont des nombres entiers d'une valeur d'au moins 2 et non supérieure à 250;
x, x' et x" ont chacun la valeur de 0 ou sont un nombre entier d'au moins 1, dans laquelle chaque nombre entier varie pour fournir au moins 30 acides aminés des motifs de séquence de répétition individuels A, A' et A", et dans laquelle le nombre entier de x' et le nombre entier de x" sont identiques ou différents du nombre entier de x;
B et B' comprennent chacun une séquence d'acides aminés d'environ 4 à environ 50 acides aminés, dans lesquelles la séquence d'acides aminés de B' est identique ou différente de la séquence d'acides aminés de B;
b et b' sont chacun un nombre entier d'une valeur de 0 à 3, dans laquelle le nombre entier de b' est identique ou différent du nombre entier de b; et
i est un nombre entier d'une valeur de 1 à 100.

3. Nanoparticule selon la revendication 2, dans laquelle le au moins un polymère de protéine à séquence de répétition est un copolymère.

4. Nanoparticule selon la revendication 3, dans laquelle le copolymère est une combinaison de motifs de soie et de motifs d'élastine.

5. Nanoparticule selon l'une quelconque des revendications 1 à 4, comprenant en outre un agent actif sélectionné parmi les protéines, les peptides, les hormones, les médicaments, les cytokines, les substances thérapeutiques, les produits cosméceutiques et neutraceutiques.

6. Nanoparticule selon la revendication 5, dans laquelle l'agent actif est une hormone.

7. Nanoparticule selon la revendication 6, dans laquelle l'hormone est sélectionnée parmi l'insuline, les facteurs de croissance, l'ACTH, l'aldostérone, la rénine, le cortisol, la DHEASE, l'oestrogène, l'hormone de croissance, l'IGF-1, l'hormone lutéinisante, la prolactine, la testostérone et l'hormone de stimulation de la thyroïde.

8. Nanoparticule selon la revendication 7, dans laquelle l'hormone est l'insuline.

9. Nanoparticule selon l'une quelconque des revendications précédentes, comprenant en outre de l'eau ou un solvant et/ou un additif sélectionné parmi les polyéthylène glycols, les polylactides, la gélatine, les polyoxomères, la lactine, les huiles végétales, l'albumine sérique humaine, les agents de durcissement et les ions multivalents.

10. Nanoparticule selon l'une quelconque des revendications précédentes, ayant une taille de 100 nanomètres à 500 nanomètres.

11. Nanoparticule selon l'une quelconque des revendications 5 à 10, dans laquelle l'agent actif peut être libéré en étant retenu à l'intérieur du polymère de protéine à séquence de répétition par des charges électrostatiques, la liaison à l'hydrogène ou l'interaction hydrophobe.

12. Nanoparticule selon l'une quelconque des revendications précédentes, pour l'utilisation dans une composition neutraceutique, cosméceutique ou thérapeutique pouvant être administrée par voie nasale ou parentérale.

13. Composition comprenant des nanoparticules selon l'une quelconque des revendications 1 à 11.

14. Procédé de fabrication d'une nanoparticule selon l'une quelconque des revendications 1 à 11, le procédé comprenant:
la fourniture d'au moins un polymère de protéine à séquence de répétition;
la soumission du au moins un polymère de protéine à séquence de répétition à une technique sélectionnée parmi la précipitation par antisolvant supercritique avec transfert de masse amélioré (SAS-EM), la précipitation par antisolvant supercritique (SAS), les solutions supercritiques à expansion rapide (RESS), la lyophilisation dans de l'azote liquide et la polymérisation en émulsion.

15. Procédé selon la revendication 14, dans lequel la fourniture d'au moins un polymère de protéine à séquence de répétition comprend la fourniture d'un copolymère et l'étape de soumission comprend la soumission du copolymère à une SAS-EM.

16. Procédé selon la revendication 14, comprenant en outre l'addition d'un agent actif au copolymère avant la soumission du copolymère à la SAS-EM et/ou l'addition d'une hormone au copolymère.
